# EUROPEAN PATENT SPECIFICATION

(11) **EP 1 866 435 B1**
(45) Date of publication and mention of the grant of the patent: **24.12.2008**
(21) Application number: 06710126.1
(22) Date of filing: 22.03.2006
(51) Int. Cl.: C12Q 1/68

(54) **POLYMORPHISM DETECTION METHOD**
POLYMORPHISMUSNACHWEISVERFAHREN
PROCEDE DE DETECTION DE POLYMORPHISME

(30) Priority: 22.03.2005 GB 0505763
(43) Date of publication of application: 19.12.2007
(73) Proprietor: Enigma Diagnostics Ltd., Salisbury, Wiltshire SP4 0JQ (GB)
(72) Inventor: SQUIRRELL, David James Enigma Diagnostics Ltd., Salisbury, Wiltshire SP4 0JQ (GB)
(74) Representative: Greaves, Carol Pauline
(86) International application number: PCT/GB2006/001014
(87) International publication number: WO 2006/100462

(56) References cited:
- WO-A-20/05003386
- US-A1- 2003 124 544
- US-A1- 2004 197 793
- TANNOUS B A ET AL: "Combined flash- and glow-type chemiluminescent reactions for high-throughput genotyping of biallelic polymorphisms" ANALYTICAL BIOCHEMISTRY, ACADEMIC PRESS, SAN DIEGO, CA, US, vol. 320, no. 2, 15 September 2003 (2003-09-15), pages 266-272, XP004447154 ISSN: 0003-2697

## Description

The present invention relates to a method for detecting specific nucleic acid sequences and in particular in identifying sequences which contain polymorphisms or mutations, as well as to kits for carrying out the assay.

A wide variety of methods are becoming available to detect particular nucleic acid sequences, and, in particular to determine the precise sequence of nucleic acids. Many of these are based upon nucleic acid amplification reactions such as the polymerase chain reaction (PCR) and the ligase chain reaction (LCR).

These methods are used in a wide variety of methods, to detect or diagnose for example diseases caused by pathogens such as bacteria and viruses. There are also particularly useful in genetic analysis or diagnosis, where the presence of a polymorphism, in particular a single nucleotide polymorphism (SNP) can give rise to a disease state, or a predisposition towards a particular disease state.

With the completion of the sequencing of the human genome, and increasing knowledge of the relationship of particular polymorphisms to particular diseases or predisposition to disease, the numbers of analyses which are being carried out for diagnostic purposes is increasing.

The ligase chain reaction is a particularly useful reaction for the detection of polymorphisms. In this reaction, a pair of complementary probes, which, when the correct template is available, hybridise next to each other on the template, are added to the sample under test. They are subjected to conditions under which such hybridisation takes place, and then ligated using a ligase enzyme, in the presence of a nucleotide which is commonly adenosine tri-phosphate (ATP), but nicotinamide adenine dinucleotide (NAD) may also be used.

A 3-step ligation event occurs during this process, as illustrated for example in an article by J.M.Pascal et al., Nature, (2004) 432,pp 473-478, resulting in the release of a molecule of adenosine monophosphate (AMP) as a by-product of each ligation event.

The ligated probes and the original template then serve as additional templates for a further round of hybridisation and ligation. This is effected by first heating the mixture so as to denature the nucleic acid strands, for example, to temperatures of from 80-90°C, suitably at about 85°C, and thereafter, cooling to a temperature at which the probes anneal and ligation occurs, for example from 40-60°C, typically at about 50°C. As subsequent cycles are carried out, the amplification but only if the correct template is present in the sample.

By using four probes, one pair corresponding to each strand in the original template molecule, which is double stranded, exponential amplification can result. In this case, in a preliminary step, the double stranded template DNA is denatured.

Furthermore, in some cases, the probes are designed so that, although they anneal close together, they are not directly adjacent each other. In these cases, a polymerase, nucleotides and other reagents necessary to allow the upstream probe to be extended across the small gap (generally of 2-3 base pairs) between the probes, is also included in the reaction mixture so as to cause the upstream probe (which functions then as a primer in an polymerase extension reaction) to extend across the gap prior to ligation. Extension will only occur if the 3' base of the upstream probe is matched to the corresponding base on the template, and so if it does not, as a result of a polymorphism or the like, then no subsequent ligation will occur.

Thereafter the product of the probe ligation can be detected by separating the products on the basis of size, for example on an electrophoretic gel, and staining the gel so as to detect them. Bands corresponding to a ligated probe will be larger than the individual probes, and so the presence of such a band will indicate that ligation has occurred and that the correct template was present in the sample.

This separation step and detection step is however slow and complex to perform. It can only be conducted in a laboratory environment and requires a substantial level of operator skill, and it requires a number of separate reaction stages.

In some instances, fluorescent dyes which associate with double stranded DNA and whose fluorescence changes when associated with double stranded DNA as compared to single stranded DNA may be added to the reaction mixture. If amplification has proceeded, then this will be detectable from the nature of the fluorescent signal from the dye. In this case however, it is necessary to measure the signal using a fluorimeter, which is a relatively complex apparatus. Fluorescent light of a particular wavelength must be supplied to the sample, and the emission signal, which is at a different wavelength measured.

WO2005/003386 discloses a method for determining the presence of a genetic element using a ligation reaction in which a by-product of the ligation is detected.

According to the present invention there is provided a method for detecting the presence or absence of a polymorphism at a particular position within a nucleic acid sequence in a sample, said method comprising
i) annealing a pair of probes to said nucleic acid sequence, said probes being designed such that they anneal adjacent each other, and only completely anneal to one form of the sequence, in a reaction mixture which is substantially free of ATP;
ii) ligating together any completely annealed pairs of probes in the presence of NAD and a nucleic acid ligase which uses NAD as a substrate;
iii) phosphorylating any AMP released by a ligation event to ATP, and
iv) detecting ATP in the resultant reaction mixture, **characterised in that** the pair of probes bind so that there is a small gap, which does not contain any adenine bases therebetween, and the reaction mixture further contains a polymerase and nucleotides suitable for extending the upstream probe across said gap, provided the 3' end of the upstream probe matches the corresponding base on the target nucleic acid.

Step (i) can be carried out in a method conventional in such reactions.

The probes bind so that there is a small gap therebetween, provided that there are no adenine bases in this gap region. The reaction mixture contains a polymerase, nucleotides other than adenine and any other reagents necessary to extend the upstream primer to close the gap and allow ligation only if the 3' end of the probe matches the corresponding base on the template. If this base does not match, then probe extension across the gap will not occur and ligation in step (ii) will not result.

Using this method, a ligation reaction in (ii) will only occur if the sample contains a sequence to which both probes bind fully.

The presence of a nucleic acid sequence which gives rise to such a match can be determined by detecting ATP, derived from the AMP released during the ligation reaction, in step (iv).

If, as a result of a mutation or difference in the nucleic acid sequence which forms the target of the assay, in particular at the position in which the probes bind, an end of one of the probes does not bind to the target, then the ligation event will not take place in step (ii). As a result no AMP is generated, and so phophorylation in step (iii) will not give rise to any ATP. The absence of ATP in the reaction mixture in step (iv) will indicate that the sample does not contain the target nucleic acid, and therefore the presence of a different form may be likely.

Nucleic acid ligases which are able to utilise NAD as well as or instead of ATP as a substrate are known. A particular example of such a ligase is *E.coli* ligase.

The AMP by-product of the ligation may be phosphorylated during step (iii) to ATP enzymatically either directly or by way of the production of adenosine diphosphate (ADP).

The one or more enzymes necessary to convert AMP produced to ATP (step (iii) above), may for example be selected from phosphoenolpyruvate synthase which produces ATP directly from AMP in the presence of phosphate and phosphoenolpyruvate, which are also added as a reagent to the reaction mixture. Alternatively, a combination of a nucleoside triphosphate-adenylate kinase and NTP will yield adenosine diphosphate (ADP), which may then be converted to ATP by inclusion or addition of an enzyme such as adenylate kinase.

Yet further examples of suitable enzymes include pyruvate phosphate dikinase such as that described by Eisaki et al, Biochim. et Biophys Acta 1431 (1999) 363-373.

By using NAD instead of ATP, which is conventionally utilised in LCR, the possibility arises that a signalling system based upon ATP detection may be used.

Various systems of this type are known. However they can generally be carried out rapidly, and give for example clear visible signals *in situ.* As a result, the ligation reaction can be determined in a homogenous manner, without the need for analysis of the sizes of the ligation products.

In particular, the ATP is detected using a bioluminescent system. Examples of the application of such detection systems are described for example in WO 96/02665.

Particularly suitable bioluminescent reagents, which react to the presence of ATP, include luciferin and luciferase, accompanied if necessary by a source of magnesium ions such as magnesium acetate. In the presence of ATP, these reagents produce a luminescent signal, which can be readily monitored for example using conventional luminometer devices. These are generally much simpler to operate and use than say a fluorimeter.

In generating a signal, these reagents regenerate an AMP molecule, which in the presence of the enzymes and/or reagents of (iii), will be reconverted back to ATP. Thus the signal builds up exponentially and so will be readily and rapidly detected. An example of such a system is described by Sakakibara et al., Analytical Biochemistry, 268, 94-101 (1999). This exponential rise in signal may mean that detection can be carried out directly, in circumstances where amplification reactions may previously have been required.

Bioluminescent signalling systems are more sensitive than fluorescent systems.

Furthermore, detection of such a reaction can be carried out simply and easily, using simple a light detector such as a camera. The use of complex equipment, such as fluorimeters and the like, necessary to detect signals in the widely used fluorescent signalling systems is thus avoided.

Suitably, the method described above forms a part of a ligase chain reaction (LCR). This amplification reaction may be conducted in the usual way, for example by cycling the reaction mixture through denaturation, and annealing/ligation temperatures. Thus, effectively steps (i) and (ii) above are repeated until the desired level of amplification has been achieved.

Suitably the reaction is conducted with two pairs of probes, one corresponding to each strand of a double stranded template nucleic acid sequence, so that amplification can proceed exponentially. In this case, the double stranded template nucleic acid is denatured prior to step (i).

Where this is the case, at least step (iv) and suitably steps (iii) and (iv) above may be carried out at the end of the reaction. This provides a good end-point detection method.

If desired however, the phosphorylating and bioluminescent reagents are present or added throughout the amplification reaction so that the progress of the reaction can be monitored. Generally speaking, the thermostability of reagents such as luciferase is not sufficient to allow it to be present throughout an amplification reaction that involves significant thermal cycling, and thus, it is suitably added at the end of each cycle, and measurements taken before the next cycle commences. However, the selection of thermostable luciferase enzymes such as those described for example in EP-A-524448, WO95/25798, WO 99/14336, WO 00/24878, WO01/31028 and WO2003/0068801 may facilitate some monitoring reaction of step (iv) to be carried out continuously through the LCR.

In particular, this may be further facilitated if the reaction conditions or reagents used in the LCR are such as to reduce or minimise the extent of the thermal cycling in terms of the range of temperatures which are required in order to achieve the desired result.

For example, it is possible to lower the denaturation temperature required by addition of reagents such as Betain (N,N,N-trimethylglycine) or increasing the volume of the solvents such as dimethylsulphoxide (DMSO) or dimethylformamide (DMF).

Addition of enzymes which destabilise duplexes such as topoisomerase or DNA gyrase may also be effective in reducing the denaturation temperatures to those which may be tolerated by luciferase enzymes. The amounts of these enzymes should be sufficient to ensure that there is an equilibrium reaction going on so that annealing and ligation is possible, if the temperature is reduced below that at which the destabilising enzymes is fully active.

In yet a further alternative, denaturation may be effected in a substantially isothermal manner, for example by applying electrochemical potentials to the reaction mixture in order to bring about a denaturation step.

Additionally, a luciferin recycling protein, which regenerates luciferin from oxyluciferin, such as those described in US Patent No. 5,814,504 and WO 03/042693 may be added to the reaction mixture, to avoid the need to continuously add luciferin to the reaction mixture.

Such information may be used then in the quantification of the target nucleic acid sequence in the sample, using algorithms etc. which are known in the art.

The reaction as described above could be carried out in a variety of conventional equipment. These include for example a Pyrosequencer (available from Pyrosequencing AB, Sweden), which is already provided with appropriate signal detection means. Alternatively, the reaction may be carried out using block heating devices as described for example in EP-A-0810030 and supplied by The Perkin-Elmer Corporation, rapid hot air thermal cyclers such as the RapidCycler™ and LightCycler™ from Idaho Technologys Inc. or other types of thermal cycler such as those described in WO98/24548.

In order to ensure that no, or at least a very low level of ATP is present in the sample before the assay, it may be preferable to carry out a pre-treatment step in which ATP is removed. This may be done for example if the sample containing the nucleic acid sequence is treated with apyrase to remove ATP, although since this may lead to some AMP production, it may be preferable to apply a deaminase, which destroys the adenosine residue. However, the bead wash steps should ensure effective separation of nucleic acids from the adenylates due to the increased binding of DNA thereto, and so the process can be carried out in the absence of any such step.

The method can be used in any applications in which LCR is currently feasible, both in detection of sequences such as microorganisms, virus or other pathogens, or in the diagnosis of disease. Suitably therefore the sample is a genetic sample.

In a further aspect the invention provides a kit for carrying out a method as described above, said kit comprising a nucleic acid ligase which uses NAD as a substrate, a phosphorylating agent, and one or more reagents able to produce a bioluminescent signal.

Suitable phosphorylating agents include enzymes such as those described above including a combination of phosphoenolpyruvate synthase, phosphate and phosphoenolpyruvate; a combination of a nucleoside triphosphate-adenylate kinase, NTP and adenylate kinase; or pyruvate phosphate dikinase.

Suitable reagents able to produce a bioluminescent reaction systems comprise luciferase and/or luciferin. Optionally, the kit may also comprise magnesium salts required to activate the luciferase/luciferin system, but which may also be used in the LCR.

The kit may further comprise a pair of probes suitable for annealing to a target nucleic acid, and preferably two pairs of probes, each pair being capable of annealing to corresponding regions on opposite strands of a target double stranded nucleic acid.

The kit may further comprise other reagents suitable for conducting LCR, for example NAD, a buffer (such as 250mM EPPS [N-2-hydroxyethyl)piperazine-N-(3-propanesulphonic acid), or potassium salts.

Additionally, it may also include apyrase enzyme or more suitably a deaminase enzyme to carry out the pre-treatment step outlined above.

The LCR element of the invention will now be particularly described by way of example with reference to the accompanying diagrammatic drawing, which illustrates schematically, a method used in the context of the method the invention.

In this reaction scheme, steps 1 to 3 represent a conventional cycle in an LCR reaction, with the exception only that NAD is used as the substrate for the ligase enzyme (NH₂-LysLig). In the first stage, the NAD becomes bound to the enzyme and disassociated to AMP (which is not available for phorphorylation at this stage). Pyrophosphate (PPi) is released by this process. In the presence of template DNA (1) having an upstream probe (2) and a downstream probe (3) hybridised thereto, the enzyme transfers its AMP to the 5' end of the downstream probe (3), which then ligates to the free hydroxy group at the 3' end of the upstream probe (2), forming a ligate (4) and releasing free AMP (Step 3).

The addition or presence of phosphorylating enzymes at this stage will result in conversion of the free AMP to ATP, which can then be detected in a conventional luciferase/luciferin bioluminescent reaction, illustrated at Step 4.

The system of the reaction therefore provides a reliable and easy to operate assay, in particular for the detection of polymorphisms, such as SNPs, in particular as set out in illustrative Example 1.

### Illustrative Example 1

### A. Sample preparation

This can be carried out on an instrument such as the Thermo Labsystem KingFisher machine.

A blood sample (200µl) is added to 300µl of 3.8M guanidium hydrochloride. This is then mixed for 1 minute at room temperature to lyse cellular material and release DNA. Promega MagneSil KF paramagnetic particles (20µl) are added (these may be stored in a well containing the first wash solution (see below) and transferred in from that well) and mixed for 2 minutes. Extracted DNA binds to the silica particles. The particles with bound DNA are then magnetically transferred into the first wash well which contains a 50% mixture of ethanol and TE buffer at pH 7.4 (TE buffer is 10mM Tris.HCl with 1mM EDTA). The beads are released and mixed with the first wash solution for 1 minute. This is repeated with two more identical wash solutions. After the 3^{rd} wash the beads are transferred into a TE buffer at pH8.3 at a temperature of >50°C with mixing for 12 minutes when the DNA is eluted from the silica matrix. The beads are then removed and disposed of in one of the wash wells and the prepared DNA used for the next step.

### Ligase Chain Reaction (LCR)

This can be performed in a block thermal cycler such as the Perkin Elmer 9600.

The DNA is mixed with an equal volume of LCR Assay Mix to give a final reaction volume of 50µl with the solution containing 20mM Tris.HCl, pH 7.6, buffer containing 100mM KCl, 10mM MgCl₂, 1mM EDTA, 10mM dithiothreitol, 10mM NAD⁺, 4µg/mL salmon sperm DNA, 15 units *T.aquaticus* ligase with the two diagnostic oligonucleotides at a concentration of 5µM each. The reaction is carried out with a thermal profile of 94°C for 1 min and 65°C for 4 minutes, repeated for 40 cycles. Bioluminescent detection of AMP formed from the NAD is then used to indicate whether or not LCR occurred. The diagnostic oligonucleotides are designed to have a melting temperature about 2°C above 65°C.

### Bioluminescent detection

The assays can be performed, at room temperature, in 100µl volumes in 96 well microtitre plates using a Labsystems Luminoskan Ascent plate luminometer from Thermo Labsystems.

The added bioluminescent assay mix contains 20units/ml adenylate kinase, 5mM acetyl phosphate, 2units/ml acetate kinase, 300ng/ml firefly luciferase and 1mM D-luciferin in 90mM Tris-HCl, 10mM MgCl₂, 1mM EDTA and 10mM dithiothreitol, to give a final pH of 7.9. Light emission is high where the diagnostic oligonucleotides end-match the complementary bases in the DNA target eg where positive detection of a single nucleotide polymorphism (SNP) is indicated.

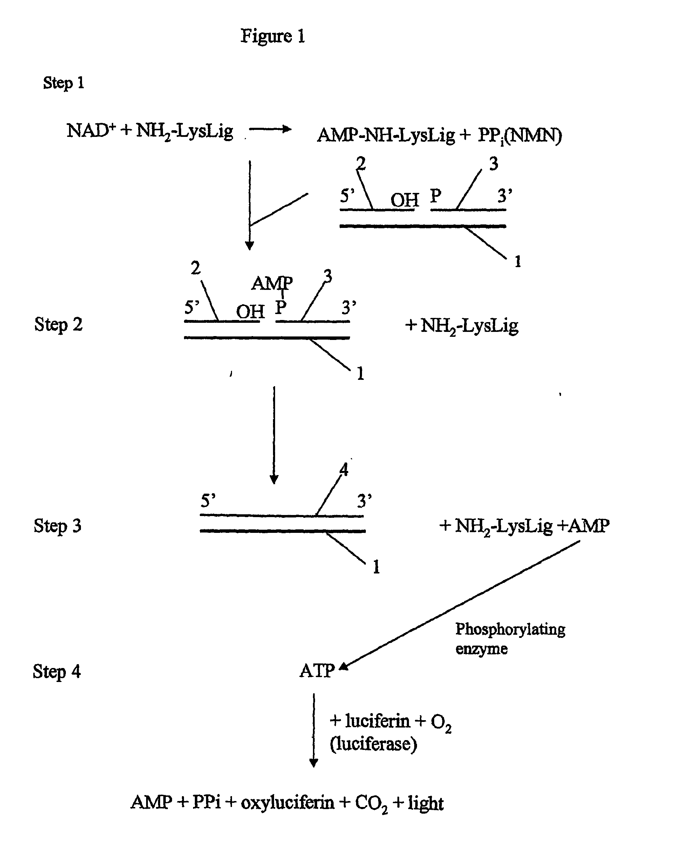

## Claims

1. A method for detecting the presence or absence of a polymorphism at a particular position within a target nucleic acid sequence in a sample, said method comprising
i) annealing a pair of probes to said nucleic acid sequence, said probes being designed such that they anneal adjacent each other, and only completely anneal to one form of the sequence, in a reaction mixture which is substantially free of ATP;
ii) ligating together any completely annealed pairs of probes in the presence of NAD and a nucleic acid ligase which uses NAD as a substrate;
iii) phosphorylating any AMP released by a ligation event to ATP, and
iv) detecting ATP in the resultant reaction mixture, **characterised in that** the pair of probes bind so that there is a small gap, which does not contain any adenine bases therebetween, and the reaction mixture further contains a polymerase and nucleotides suitable for extending the upstream probe across said gap, provided the 3' end of the upstream probe matches the corresponding base on the target nucleic acid.

2. A method according to claim 1 wherein ATP is detected using a bioluminescent system.

3. A method according to claim 2 wherein the bioluminescent system is the luciferase/luciferin system.

4. A method according to any one of the preceding claims wherein as a pre-treatment step, the sample containing the nucleic acid sequence is treated to remove ATP therefrom.

5. A method according to claim 4 wherein the sample is a genetic sample.

6. A method according to any one of the preceding claims wherein the nucleic acid ligase is a thermostable ligase, and the method forms part of a ligase chain reaction.

7. A method according to any one of the preceding claims wherein two pairs of probes are used, one pair corresponding to each strand of a double stranded target nucleic acid.

8. A kit for carrying out a method according to any one of the preceding claims, said kit comprising a nucleic acid ligase which uses NAD as a substrate, a phosphorylating agent, one or more reagents able to produce a bioluminescent signal, a pair of probes suitable for annealing to a target nucleic acid so that there is a small gap, which does not contain any adenine bases therebetween and a polymerase.

9. A kit according to claim 8 wherein the phosphorylating agent is a combination of phosphoenolpyruvate synthase, phosphate and phosphoenolpyruvate; a combination of a nucleoside triphosphate-adenylate kinase, NTP and adenylate kinase; or pyruvate phosphate dikinase.

10. A kit according to claim 8 or claim 9 wherein the one or more reagents able to produce a bioluminescent signal comprise luciferase and/or luciferin.

11. A kit according to any one of claims 8 to 10 which comprises two pairs of probes, each pair being capable of annealing to corresponding regions on opposite strands of a target double stranded nucleic acid.

12. A kit according to any one of claims 8 to 11 which further comprises magnesium salts.

13. A kit according to any one of claims 8 to 12 which further comprises one or more of NAD, a buffer, or potassium salts.

14. A kit according to any one of claims 8 to 13 which further includes an deaminase enzyme.

## Patentansprüche

1. Verfahren zum Nachweis des Vorhandenseins oder Nichtvorhandenseins eines Polymorphismus an einer bestimmten Position innerhalb einer Ziel-Nucleinsäuresequenz in einer Probe, umfassend
i) Annealen eines Paars von Sonden an die Nucleinsäuresequenz in einem Reaktionsgemisch, das im Wesentlichen frei ist von ATP, wobei die Sonden so gestaltet sind, dass sie einander benachbart annealen und nur vollständig an eine Form der Sequenz annealen;
(ii) Ligasieren von vollständig annealten Paaren von Sonden in der Gegenwart von NAD und einer Nucleinsäure-Ligase, die NAD als Substrat verwendet;
iii) Phosphorylieren von AMP, das bei einem Ligationsvorgang zu ATP freigesetzt wurde;
iv) Nachweisen von ATP in dem erhaltenen Reaktionsgemisch,
**dadurch gekennzeichnet, dass** das Paar von Sonden derart bindet, dass ein kleiner Spalt vorhanden ist, der dazwischen keinerlei Adeninbasen enthält und dass das Reaktionsgemisch außerdem eine Polymerase und Nucleotide enthält, die zum Verlängern der stromaufwärtigen Sonde über den Spalt geeignet sind, vorausgesetzt dass das 3'-Ende der stromaufwärtigen Sonde mit der entsprechenden Base auf der Ziel-Nucleinsäure paart.

2. Verfahren nach Anspruch 1, worin ATP unter Verwendung eines Biolumineszenz-Systems nachgewiesen wird.

3. Verfahren nach Anspruch 2, worin das Biolumineszenz-System das Luciferase/Luciferin-System ist.

4. Verfahren nach irgendeinem der vorhergehenden Ansprüche, worin als Vorbehandlungsschritt die Probe, welche die Nucleinsäuresequenz enthält, behandelt wird, um ATP daraus zu entfernen.

5. Verfahren nach Anspruch 4, worin die Probe eine genetische Probe ist.

6. Verfahren nach irgendeinem der vorhergehenden Ansprüche, worin die Nucleinsäure-Ligase eine thermostabile Ligase ist und das Verfahren Teil einer Ligase-Kettenreaktion bildet.

7. Verfahren nach irgendeinem der vorhergehenden Ansprüche, worin zwei Paare von Sonden verwendet werden, wobei ein Paar jedem Strang einer doppelsträngigen Ziel-Nucleinsäure entspricht.

8. Kit zur Durchführung eines Verfahrens nach irgendeinem der vorhergehenden Ansprüche, wobei das Kit umfasst eine Nucleinsäure-Ligase, die NAD als Substrat verwendet, ein Phosphorylierungsmittel, ein oder mehrere Reagentien, die zur Erzeugung eines Biolumineszenz-Signals geeignet sind, ein Paar von Sonden, die zum Annealen an eine Ziel-Nucleinsäure geeignet sind, so dass ein kleiner Spalt entsteht, der keinerlei Adeninbasen dazwischen enthält, und eine Polymerase.

9. Kit nach Anspruch 8, worin das Phosphorylierungsmittel eine Kombination von Phosphoenolpyruvat-Synthase, Phosphat und Phosphoenolpyruvat; eine Kombination einer Nucleosidtriphosphat-Adenylat-Kinase, NTP und Adenylat-Kinase; oder Pyruvatphosphat-Dikinase ist.

10. Kit nach Anspruch 8 oder Anspruch 9, worin die ein oder mehreren Reagentien, die zur Erzeugung eines Biolumineszenz-Signals geeignet sind, Luciferase und/oder Luciferin umfassen.

11. Kit nach irgendeinem der Ansprüche 8 bis 10, welches zwei Paar von Sonden umfasst, wobei jedes Paar fähig ist, an entsprechende Regionen auf gegenüberliegenden Strängen einer doppelsträngigen Ziel-Nucleinsäure zu annealen.

12. Kit nach irgendeinem der Ansprüche 8 bis 11, welches ferner Magnesiumsalze umfasst.

13. Kit nach irgendeinem der Ansprüche 8 bis 12, welches ferner ein oder mehrere von NAD, einem Puffer oder Kaliumsalzen umfasst.

14. Kit nach irgendeinem der Ansprüche 8 bis 13, welches ferner ein Deaminase-Enzym umfasst.

## Revendications

1. Procédé de détection de la présence ou de l'absence d'un polymorphisme au niveau d'une position particulière au sein d'une séquence d'acide nucléique cible dans un échantillon, ledit procédé comprenant
i) l'hybridation d'une paire de sondes à ladite séquence d'acide nucléique, lesdites sondes étant conçues de telle manière qu'elles s'hybrident adjacentes l'une à l'autre, et ne s'hybrident complètement qu'à une forme de la séquence, dans un mélange réactionnel qui est pratiquement dépourvu d'ATP ;
ii) la ligature ensemble de toutes les paires de sondes complètement hybridées en présence de NAD et d'une acide nucléique ligase qui utilise le NAD comme substrat ;
iii) la phosphorylation de tout AMP libéré par un événement de ligature à l'ATP, et
iv) la détection de l'ATP dans le mélange réactionnel résultant, **caractérisée en ce que** la paire de sondes se lie de telle manière qu'il y existe une petite brèche, qui ne contient aucune base d'adénine entre celles-ci, et le mélange réactionnel contient en outre une polymérase et des nucléotides appropriés pour étendre la sonde an amont en travers de ladite brèche, à condition que l'extrémité 3' de la sonde en amont corresponde à la base correspondante sur l'acide nucléique cible.

2. Procédé selon la revendication 1, dans lequel l'ATP est détecté en utilisant un système bioluminescent.

3. Procédé selon la revendication 2, dans lequel le système bioluminescent est le système de luciférase/ luciférine.

4. Procédé selon l'une quelconque des revendications précédentes, dans lequel comme étape de prétraitement, l'échantillon contenant la séquence d'acide nucléique est traité pour en éliminer l'ATP.

5. Procédé selon la revendication 4, dans lequel l'échantillon est un échantillon génétique.

6. Procédé selon l'une quelconque des revendications précédentes, dans lequel l'acide nucléique ligase est une ligase thermostable, et le procédé forme une partie d'une réaction en chaîne de la ligase.

7. Procédé selon l'une quelconque des revendications précédentes, dans lequel deux paires de sondes sont utilisées, une paire correspondant à chaque brin d'un acide nucléique cible double brin.

8. Kit de réalisation d'un procédé selon l'une quelconque des revendications précédentes, ledit kit comprenant une acide nucléique ligase qui utilise le NAD comme substrat, un agent de phosphorylation, un ou plusieurs réactifs capables de produire un signal bioluminescent, une paire de sondes appropriées pour s'hybrider à un acide nucléique cible de manière à ce qu'il existe une petite brèche qui ne contient aucune base d'adénine entre celles-ci et une polymérase.

9. Kit selon la revendication 8, dans lequel l'agent de phosphorylation est une combinaison de phosphoénol-pyruvate synthase, de phosphate et de phosphoénol-pyruvate ; une combinaison d'un nucléoside triphosphate-adénylate kinase, de NTP et d'adénylate kinase ; ou la pyruvate phosphate dikinase.

10. Kit selon la revendication 8 ou la revendication 9, dans lequel un ou plusieurs réactifs capables de produire un signal bioluminescent comprennent la luciférase et/ou la luciférine.

11. Kit selon l'une quelconque des revendications 8 à 10, qui comprend deux paires de sondes, chaque paire étant capable de s'hybrider aux régions correspondantes sur les brins opposés d'un acide nucléique double brin cible.

12. Kit selon l'une quelconque des revendications 8 à 11, qui comprend en outre des sels de magnésium.

13. Kit selon l'une quelconque des revendications 8 à 12, qui comprend en outre un ou plusieurs du NAD, d'un tampon ou des sels de potassium.

14. Kit selon l'une quelconque des revendications 8 à 13, qui comprend en outre une enzyme désaminase.
